# EUROPEAN PATENT SPECIFICATION

(11) **EP 2 470 519 B1**
(45) Date of publication and mention of the grant of the patent: **17.12.2014**
(21) Application number: 10811030.5
(22) Date of filing: 27.08.2010
(51) Int. Cl.: C07D 307/86, A61K 31/343, A61P 27/02

(54) **TREATMENT OF MACULAR DEGENERATION**
BEHANDLUNG VON MAKULADEGENERATION
TRAITEMENT DE LA DÉGÉNÉRESCENCE MACULAIRE

(30) Priority: 27.08.2009 AU 2009904097
(43) Date of publication of application: 04.07.2012
(73) Proprietor: Bionomics Limited, Thebarton, S.A. 5031 (AU)
(72) Inventor: KREMMIDIOTIS, Gabriel, Flagstaff Hill South Australia 5159 (AU)
(74) Representative: Kling, Simone
(86) International application number: PCT/AU2010/001108
(87) International publication number: WO 2011/022781

(56) References cited:
- WO-A1-2006/084338
- WO-A1-2006/084338
- WO-A1-2007/087684
- WO-A1-2007/087684
- WO-A1-2009/043104
- GILL, G. S. ET AL.: 'An efficient synthesis and Substitution of 3-aroyl-2- bromobenzo[b]furans' J. ORGANIC CHEM. vol. 73, no. 3, 2008, pages 1131 - 1134, XP008159974

## Description

### Field of the Invention

The present invention relates to compounds and pharmaceutical compositions for use in the treatment of macular degeneration (MD) and in particular age-related macular degeneration (AMD). Further, the present invention provides for the use of specific benzofuran based compounds in the manufacture of a medicament for treating MD and AMD.

### Background of the Invention

Macular degeneration (MD) is a medical condition that usually occurs in the elderly (also referred to as age-related macular degeneration (AMD)) and may result in a loss of vision in the macular due to retinal damage. MD and AMD are complex disorders that involve molecular derangement in or near Bruch's membrane, the multilaminar sandwich of extracellular matrix that lies between the retinal pigment epithelium and the anastomotic blood supply of the outer retinal known as choriocapillaris. This derangement can lead to the growth of new blood vessels (commonly referred to as choroidal neovascularization) from the choriocapillaris through Bruch's membrane and into the subretinal pigment epithelial or subretinal space. This neovascular complication can be so damaging to the structure and function of the retina it results in blindness attributable to the disease.

Known treatments for MD or AMD involving choroidal neovascularization include anti-VEGF (Vascular Endothelial Growth Factor) agents that cause regression of abnormal blood vessel growth and improvement of vision when injected directly into the vitreous humour of the eye. Examples of these agents include the monoclonal antibodies to VEGF, ranibizumab (marketed as "Lucentis"), bevacizumab (marketed as "Avastin") and pegatanib (marketed as "Macugen"). Treatments involving the use of these drugs are often expensive and often not effacious. There is therefore a clear need to identify alternative beneficial cellular targets for the treatment of AMD and develop suitable therapies around these targets.

The international patent applications WO 2007/087684 A1 and WO 2006/084338 A1 disclose BNC105, a compound of formula (I), and its use for the treatment of diseases linked to tubulin polymerization.

### Summary of the Invention

The present invention is now predicated on the discovery that a particular benzofuran based compound displays superior selectivity for inhibiting growth of angiogenic endothelial cells, and that this compound has surprising advantages in the treatment of macular degeneration.

In oneaspect of the present invention there is provided a pharmaceutical composition comprising an effective amount of a compound of formula (I) or a pharmaceutically acceptable salt, solvate or C-7 disodium phosphate ester prodrug thereof: 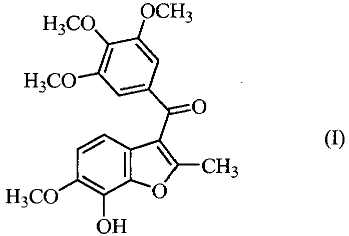 optionally in combination with a pharmaceutically acceptable carrier, excipient or diluent, for use in the treatment of macular degeneration (MD).

In another aspect of the present invention there is provided a use of a compound of formula (I) or a pharmaceutically acceptable salt, solvate or C-7 disodium phosphate ester prodrug thereof: 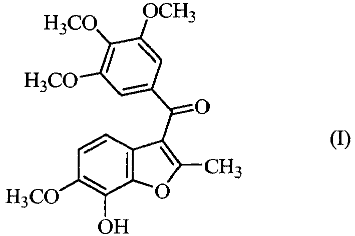 in the manufacture of a medicament for treating macular degeneration (MD).

In another aspect of the present invention there is provided a compound of formula (I) or a pharmaceutically acceptable salt, solvate or C-7 disodium phosphate ester prodrug thereof: 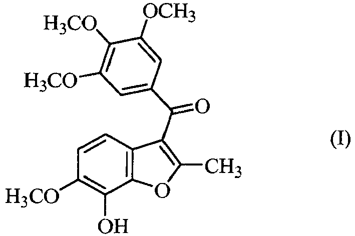 for use in the treatment of macular degeneration (MD).

### Brief Description of the Figures

**Figure 1** depicts a graph of cell viability/proliferation relative to control against concentration of CA4 (nM) in relation to proliferating and quiescent endothelial cells.

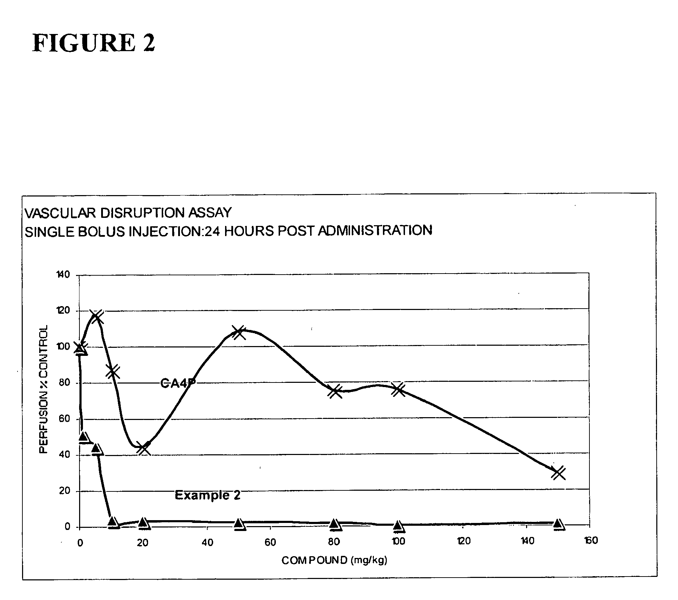

**Figure 2** depicts a graph of % perfusion control against an amount of compound (mg/kg) in relation to comparative levels of vascular shutdown (reduction in tumour perfusion) between Combretastatin A4 phosphate (CA4P) and compound Example 2 for use according to the present invention.

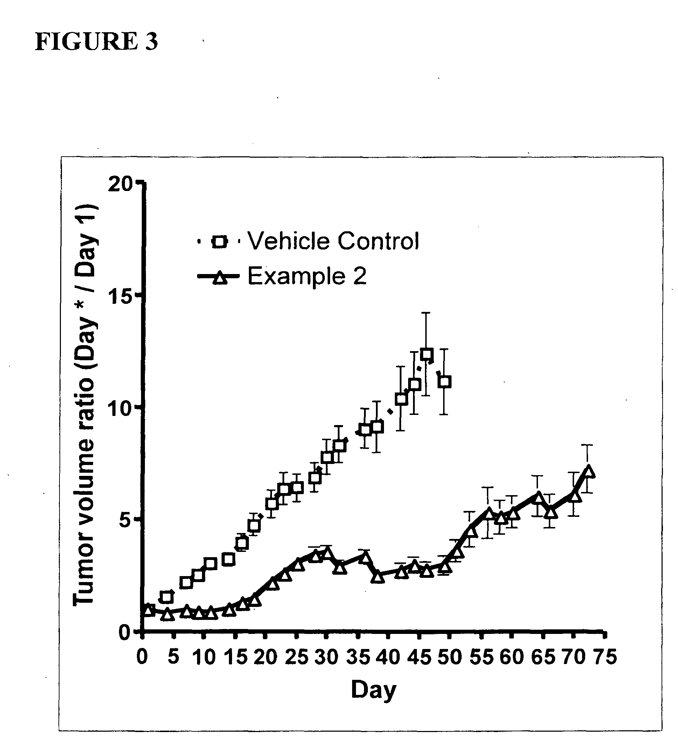

**Figure 3** depicts a graph of tumour volume ratio (Day*/Day 1) against time (Days) as a measure of tumour growth inhibition by compound Example 2 in Balb/c nu/nu mice bearing MDA-MB-231 orthotopic breast solid tumors.
**Figure 4** depicts the selectivity of compound Example 2 for angiogenic endothelium as compared to CA4. The ratio of IC50 activity in terms of quiescent/activated endothelial cells in culture is shown.
**Figure 5** depicts endothelial cell capillary formation on Matrigel is more significantly inhibited by compound Example 2 than CA4. DMSO vehicle was included in the analysis as a negative control. Cells were plated at 25,000/well in 96-well plates and cultured for 22 hrs.
**Figure 6** depicts the disruptive activity exhibited by compound Example 2 and CA4 on stable pre-formed HUVEC capillary tubes on Matrigel. Pre-formed capillaries were exposed to compound Example 2 and CA4 at concentrations that were found to inhibit capillary formation. HUVEC capillaries immediately after administration of (A) compound Example 2, (C) CA4; and HUVEC capillaries 5 hrs after (B) compound Example 2, (D) CA4.
**Figure 7** depicts disruption of endothelial cell cytoskeleton by compound Example 2. Endothelial cells were cultured in the presence of 10nM compound Example 2 or DMSO control, and after 20 mins washed, fixed and stained with actin.

### Description of the Preferred Embodiments

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Tubulin is one potential target for treating disease states that are dependent or result from the abnormal formation of blood vessels (neovascularisation) including ocular myopathy and cancer. Tubulin is composed of a heterodimer of two related proteins called α and β tubulin. Tubulin polymerises to form structures called microtubules. Compounds that inhibit tubulin's ability to polymerise to form microtubules interrupt cell division which is dependent on the formation of microtubules to form mitotic spindles. Examples of such compounds include the Vinca alkaloids such as vincristine and vinblastine.

For these antimitotic agents, selectivity for diseased versus non-diseased tissue is based on relative rates of proliferation, where the diseased tissue more rapidly proliferates. Accordingly, diseased tissue is generally more sensitive to the effect of these agents because it is more likely to be in a state of mitosis which is the stage of a cell's life cycle affected by agents that target tubulin. Unfortunately however, a number of normal, healthy tissues also have quite high rates of proliferation (for example hair follicles and the lining of the gastro-intestinal tract) and accordingly, these tissues can be damaged during chemotherapy with these agents.

In the case of ocular myopathy and cancer, the cytoskeleton of vascular endothelial cells is disrupted through depolymerisation of microtubles, which results from inhibiting the polymerisation of tubulin to form microtubules. Microtubule length is dependent on the rate of depolymerisation versus polymerisation. Depolymerising microtubles through inhibition of polymerisation leads to a change in endothelial cell morphology, which then causes a blockage or shutdown in blood flow. In the case of cancerous tumours, blood flow to the diseased tissue is stopped, depriving the tumour of oxygen and nutrients leading to necrotic cell death. Neovascular systems are more sensitive to these agents because they are more dependent on microtubule cytoskeletons than normal, healthy, vascular endothelial cells which are also supported by actin based cytoskeletal structures. For a number of tubulin polymerisation inhibitors (TPIs) that target the colchicine binding site of tubulin, the vascular targeting modality can be achieved at a lower *in vivo* concentration than the antiproliferative modality. In theory though, agents that target the colchicine binding domain of tubulin are potentially dual mode agents (ie. antimitotic and antivascular).

One of the most potent inhibitors of tubulin polymerisation that binds to the colchicine binding domain of tubulin is the *cis*-stilbene, combretastatin A4 (CA4). Due to its insolubility CA4 is administered as its prodrug equivalent combretastatin A4 disodium phosphate (CA4P), where the phosphate is rapidly *cleaved in vivo.* CA4P is currently undergoing phase II and III clinical trials and is one of the most advanced vascular disrupting agents being trialed. In view of some of the draw-backs associated with CA4P, such as, instability (can isomerise to the inactive *trans*-stilbene), toxicity and rapid clearance, a number of synthetic groups have sought to prepare more stable analogues that could be designed to exhibit an improved therapeutic index and exhibit improved pharmacokinetics. Recently, a number of TPIs have been identified that contain the benzofuran, indole or benzothiophene ring systems (see for example, WO 1998/39323, WO 2001/19794 and WO 2001/68654) or chromene and dihydronapthalene ring systems (see for example, WO 2005/113532, and WO 1998/39323). Such ring systems are quite stable and should over come the stability issues associated with CA4P.

The present invention is based on the discovery that a particularly substituted benzofuran compound of formula (I), 2-Methyl-7-hydroxy-3-(3,4,5-trimethoxybenzoyl)-6-methoxybenzofuran, or a pharmaceutically acceptable salt, solvate or prodrug thereof: 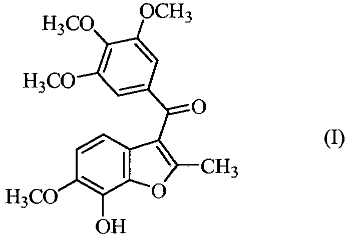 shows surprising superior selectivity and potentcy for activated angiogenic endothelial cells as compared to other tubulin polymerisation inhibitors such as similarly substituted benzofuran derivatives and combrestatin (CA4), and that the compound has specific utility in the treatment of MD.

In this specification, the term "macular degeneration" or "MD" is intended to include age-related macular degeneration (AMD), but does not exclude macular degeneration in patients who are not elderly. Accordingly, AMD and MD as referred to herein may be used interchangeably. In the context of the present invention it will be appreciated that the term "macular degeneration" particularly refers to "wet" MD also known as neovascular or exudative AMD.

The compound of formula (I) (2-Methyl-7-hydroxy-3-(3,4,5-trimethoxybenzoyl)-6-methoxybenzofuran) can be prepared by standard methodology such as that described in Example 1.

Said compound for use according to the invention can be administered to a subject as a pharmaceutically acceptable salt thereof. Suitable pharmaceutically acceptable salts include, but are not limited to salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, maleic, citric, lactic, mucic, gluconic, benzoic, succinic, oxalic, phenylacetic, methanesulphonic, toluenesulphonic, benezenesulphonic, salicyclic sulphanilic, aspartic, glutamic, edetic, stearic, palmitic, oleic, lauric, pantothenic, tannic, ascorbic and valeric acids.

Base salts include, but are not limited to, those formed with pharmaceutically acceptable cations, such as sodium, potassium, lithium, calcium, magnesium, ammonium and alkylammonium. Are included in particular cationic salts eg sodium or potassium salts, or alkyl esters (eg methyl, ethyl) of the phosphate group.

Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

The compound for use according to the invention can be administered to a subject in the form of a pharmaceutically acceptable pro-drug. The term "pro-drug" is used in its broadest sense and encompasses those derivatives that are *converted in vivo* to the compound for use according to the invention. Such derivatives would readily occur to those skilled in the art, and include, for example, compounds where a free hydroxy group (for instance at the C-7 position) is converted into an ester, such as an acetate or phosphate ester. Procedures for esterifying (eg. acylating), the compounds for use according to the invention are well known in the art and may include treatment of the compound with an appropriate carboxylic acid, anhydride or chloride in the presence of a suitable catalyst or base. A particularly preferred prodrug is a disodium phosphate ester. The disodium phosphate ester of the compound for use according to the invention may be useful in increasing the solubility of the compounds. This would, for instance, allow for delivery of the compound in a benign vehicle like saline. The disodium phosphate ester may be prepared in accordance with the methodology described in Pettit, G. R., et al, Anticancer Drug Des., 1995, 10, 299. Other texts which generally describe prodrugs (and the preparation thereof) include: Design of Prodrugs, 1985, H. Bundgaard (Elsevier); The Practice of Medicinal Chemistry, 1996, Camille G. Wermuth et al., Chapter 31 (Academic Press); and A Textbook of Drug Design and Development, 1991, Bundgaard et al., Chapter 5, (Harwood Academic Publishers).

The compound for use according to the invention may be in crystalline form either as the free compound or as a solvate (e.g. hydrate) and it is intended that both forms are within the scope of the present invention. Methods of solvation are generally known within the art.

The compound of formula (I) (hereinafter also referred to as "the compound for use according to the invention") is observed to be potent tubulin polymerisation inhibitor (TPI). TPI compounds have been shown to be important in the treatment of cancers primarily as a result of their capacity to selectively shut down blood flow through a tumour. Compounds that inhibit tumour blood flow are generally referred to as vascular disrupting agents (VDAs) (Tozer, G. M.; Kanthou, C.; Baguley, B. C. Nature Rev., Vol. 5, 2005, 423). TPIs are known vascular disruption agents because they inhibit a certain cell signalling pathway associated with microtubules, leading to interference in the regulation of the cytoskeleton of the endothelial cells that line the blood vessels of the tumour. As a result, these usually flat cells become more rounded, ultimately occluding blood flow through the vessels.

In wet AMD, VEGF-A is believed to play a significant role in the formation of blood vessels that grow abnormaly and leak beneath the macula. The constant exposure of endothelial cells to pro-angiogenic factors, such as VEGF-A, result in the formation of immature, semi-differentiated and fragile blood vessels which have a tendency to leak and bleed. Exposure of these cells to TPI agents may lead to occlusion of such vessels and moreoever suppress further angiogenesis and neovascularisation. However, most known TPIs do not distinguish between activated and quiescent endothelial cells. For example, Combretastatin A4 (CA4), a known TPI, is equally selective against quiescent (normal) endothelial cells and activated (angiogenic) endothelial cells. It is therefore possible that, when used in the treatment of AMD, CA4 would target normal endothelial cells, thereby disrupting the normal vasculature of the eye and cause irreversible damage to a patient's sight. On the other hand, the compound for use according to the invention displays a surprisingly greater selectivity and potentcy for actived (angiogenic) endothelial cells as compared to quiescent (normal) cells. This discovery has important therapeutic application for the treatment of macular degeneration. It means that the compound for use according to the invention, when formulated as a suitable pharamaceutical and administered to a patient suffering from macular degeneration, will be more potent against activated (angiogenic) endothelial cells at much lower doses due to its higher selectivity (ie. it is thus expected to have a wider therapeutic window than compounds or agents that do not distinguish between activated and quiescent endothelial cells).

In this specification, the term "activated" as used in the context of "activated endothelial cells" or "activated (angiogenic) endothelial cells" is intended to mean endothelial cells that are in an angiogenic and/or proliferative state, whereas the term "quiescent" as used in the context of "quiescent endothelial cells" or "quiescent (normal) endothelial cells" is intended to mean those endothelial cells that are part of stable, mature capillaries and are mitotically inert.

As previously indicated, the present invention is based on the discovery that a compound of formula (I) as defined herein displays considerable higher cytotoxic and anti-angiogenic activity towards activated (angiogenic) endothelial cells as compared to quiescent (non-angiogenic) endothelial cells. This selectivity means the compound of formula (I), as well as pharmaceutically acceptable salt, solvate or prodrug thereof, is particularly well suited for therapeutic application to patients with macular degeneration it is able to distinguish between activated endothelial cells from quiescent endothelial cells, and inhibit proliferation of only activated cells.

The present invention also provides a pharmaceutical composition comprising an effective amount of a compound of formula (I) or a pharmaceutically acceptable salt, solvate or C-7 disodium phosphate ester prodrug thereof, optionally in combination with a pharmaceutically acceptable carrier, excipient or diluent, for use in the treatment of macular degeneration (MD).

There is also provided a use of a therapeutically effective amount of a compound of formula (I) or a pharmaceutically acceptable salt, solvate or prodrug thereof, for the manufacture of a medicament for treating macular degeneration (MD).

The present invention also provides a compound of formula (I) or a pharmaceutically acceptable salt, solvate or prodrug thereof for use in the treatment of macular degeneration (MD).

The disease pathology of age-related macular degeneration can be multi-factorial. In the treatment of macular degeneration, different therapies may be combined *(i.e.* combination therapies). The term "therapeutic agent", "other therapeutic agent", "another therapeutic agent", "second therapeutic agent" and the like, as used herein is intended to include other therapeutic compounds or treatments which may be used in combination with the compound for use according to the present invention. These other therapeutic compounds or treatments include, but are not limited to, angiogenesis inhibitors, vascular endothelial growth factor (VEGF) inhibitors, fibroblast growth factor-1 (FGF-1) inhibitors, antihypertensive agents, receptor tyrosine kinase inhibitors, corticosteroids, mammalian/mechanistic targets of rapamycin (mTOR) inhibitors, non-steroidal antiinflammatory drugs (NSAIDs), complement inhibitors, short interfering RNA molecules (siRNA) against certain targets, monoclonal antibodies against certain targets, vasodilators, immunomodulators, tyrosine inhibitors, tumour necrosis factor (TNF) inhibitors, blood flow inhibitors, photodynamic therapy, laser photocoagulation, as well as other MD or AMD specific treatments. For example, a compound of formula (I) or a pharmaceutically acceptable salt, solvate or prodrug may be administered in combination with one or more of the compounds or treatments listed in Table 1.

The therapeutic compounds or treatments used in such combination therapies may be administered together with a compound of formula (I) or a pharmaceutically acceptable salt, solvate or prodrug, one after the other, separately in one combined unit dosage or in separate unit dosage forms.

Examples of therapeutic agents for use in combination therapies according to the present invention include VEGF inhibitors such as avastin, lucentis and/or macugen. In certain embodiments, a compound of formula (I) or a pharmaceutically acceptable salt, solvate or prodrug is administered to a patient in need thereof in combination with avastin; or a compound of formula (I) or a pharmaceutically acceptable salt, solvate or prodrug is administered to a patient in need thereof in combination with lucentis; or a compound of formula (I) or a pharmaceutically acceptable salt, solvate or prodrug is administered to a patient in need thereof in combination with macugen. Again, the VEGF inhibitors including avastin, lucentis and/or macugen used in such combination therapies may be administered together, one after the other, separately in one combined unit dosage or in separate unit dosage forms.

An example of a use in combination therapies according to the present invention is photodynamic therapy. In an embodiment, a compound of formula (I) or a pharmaceutically acceptable salt, solvate or prodrug is administered to a patient in need thereof in combination with photodynamic therapy (PDT). In this respect, the patient may be subjected to the PDT before, at the same time or after a compound of formula (I) or a pharmaceutically acceptable salt, solvate or prodrug has been administered to the patient. PDT is an art known technique which would be familiar to the skilled person.

**Table 1: Examples of therapeutic compounds or treatments for use in combination therapies with the compound of formula (I)**

| **Category** | **Compound/Treatment** |
|---|---|
| Anti-VEGF | Avastin Intravitreal Injection |
| Anti-VEGF | Lucentis (Ranibizumab) |
| Anti-VEGF aptamer | Macugen (Pegaptamib) |
| Recombinant fusion protein VEGF receptor | KH902 |
| Receptor tyrosine kinase inhibitor | Pazopanib |
| Receptor tyrosine kinase inhibitor | Imatinib |
| VEGFR tyrosine kinase inhibitor | AG-013958 |
| VEGF Receptor tyrosine kinase inhibitor | PTK787 |
| Receptor tyrosine kinase inhibitor | AL-39324 |
| Multi tyrosine inhibitor | TG100801 |
| mTOR inhibitor | Everolimus |
| mTOR inhibitor | Sirolimus |
| mTORC1/2 + PI3K inhibitor | Palomid 529 |
| Corticosteroid | Triamcinolone acetonide (Kenacort) |
| Corticosteroid | IBI-20089C (Triamcinolone acetonide intravitreal injection) |
| Corticosteroid | Fluocinolone |
| Corticosteroid | Nova63035 |
| Anti-angiogenic | Squalamine lactate /MSI-1256F |
| Anti-angiogenesis | ORA102 |
| Immunomodulator | Copaxone (Glatiramer acetate) |
| Angiogenesis inhibitor | Retaane (Anecortave) |
| NSAID | Celecoxib (Celebrex) |
| TNF inhibitor | Adalimumab |
| | Infliximab |
| Antihypertensive | Mecamylamine (ATG003) |
| Anti-IL2 therapy | Daclizumab |
| vasodilator | Alprostadil (prostaglandin E1) |
| Integrin inhibitor (monoclonal) | Volociximab |
| Integrin alpha5 beta 1 antagonist | JSM6427 |
| Complement inhibitor | Pot4 |
| Complement inhibitor | AL-78898A |
| Monoclonal antibody against S1P | iSONEP |
| Monoclonal antibody ILlbeta | ACZ885 |
| Anti-CD11 | Efalizumab |
| siRNA to RTP-801 gene | PF-04523655 |
| siRNA | AGN211745 |
| siRNA VEGF | Bevasoranib |
| Anti-C5 aptamer | ARC1905 |
| Anti-PDGF pegylated aptamer | E10030 |
| Anti VEGF aptamer | EYE001 |
| Treatment of vitreomacular adhesion | Microplasmin |
| Visual cycle modulator | ACU-4429 |
| Inhibitor of alcohol dehygrogenase | 4-Methylpyrazol |
| Carotenoid: supplement to protect against AMD | Lutein |
| Blood flow inhibitor | MC-1101 |
| Photodynamic therapy | Visudyne therapy |
| Laser photocoagulation | |

The compound for use according to the invention, or a pharmaceutically acceptable salt, solvate or C-7 disodium phosphate ester prodrug thereof is administered to the patient in a therapeutically effective amount. As used herein, a therapeutically effective amount is intended to include at least partially attaining the desired effect, or delaying the onset of, or inhibiting the progression of, or halting or reversing altogether the onset or progression of macular degeneration.

As used herein, the term "effective amount" relates to an amount of compound which, when administered according to a desired dosing regimen, provides the desired therapeutic activity. Dosing may occur at intervals of minutes, hours, days, weeks, months or years or continuously over any one of these periods. Suitable dosages may lie within the range of about 0.1 ng per kg of body weight to 1 g per kg of body weight per dosage, such as is in the range of 1 mg to 1 g per kg of body weight per dosage. In one embodiment, the dosage may be in the range of 1 mg to 500 mg per kg of body weight per dosage. In another embodiment, the dosage may be in the range of 1 mg to 250 mg per kg of body weight per dosage. In yet another embodiment, the dosage may be in the range of 1 mg to 100 mg per kg of body weight per dosage, such as up to 50 mg per body weight per dosage.

Suitable dosage amounts and dosing regimens can be determined by the attending physician and may depend on the severity of the condition as well as the general age, health and weight of the patient to be treated.

The compound for use according to the invention may be administered in a single dose or a series of doses. While it is possible for the active ingredient to be administered alone, it is preferable to present it as a composition, preferably as a pharmaceutical composition. The formulation of such compositions is well known to those skilled in the art. The composition may contain any suitable carriers, diluents or excipients. These include all conventional solvents, dispersion media, fillers, solid carriers, coatings, antifungal and antibacterial agents, dermal penetration agents, surfactants, isotonic and absorption agents and the like. It will be understood that the compositions for use according to the invention may also include other supplementary physiologically active agents.

The carrier must be pharmaceutically "acceptable" in the sense of being compatible with the other ingredients of the composition and not injurious to the patient. The compositions may conveniently be presented in unit dosage form and may be prepared by any methods well known in the art of pharmacy. Such methods include the step of bringing into association the active ingredient with the carrier which constitutes one or more accessory ingredients. In general, the compositions are prepared by uniformly and intimately bringing into association the active ingredient with liquid carriers or finely divided solid carriers or both, and then if necessary shaping the product.

In an embodiment, the compound of formula (I) or a pharmaceutically acceptable salt, solvate or prodrug is injected directly to the eye, and in particular the vitreous of the eye. The compound or compositions for use according to the invention can be administered to the vitreous of the eye using any intravitreal or transscleral administration technique. For example, the compound or compositions can be administered to the vitreous of the eye by intravitreal injection. Intravitreal injection typically involves administering a compound for use according to the invention or a pharmaceutically acceptable salt, solvate or prodrug in a total amount between 0.1 ng to 10 mg per dose.

Injectables for such use can be prepared in conventional forms, either as a liquid solution or suspension or in a solid form suitable for preparation as a solution or suspension in a liquid prior to injection, or as an emulsion. Carriers can include, for example, water, saline (e.g., normal saline (NS), phosphate-buffered saline (PBS), balanced saline solution (BSS)), sodium lactate Ringer's solution, dextrose, glycerol, ethanol, and the like; and if desired, minor amounts of auxiliary substances, such as wetting or emulsifying agents, buffers, and the like can be added. Proper fluidity can be maintained, for example, by using a coating such as lecithin, by maintaining the required particle size in the case of dispersion and by using surfactants. By way of example, the compound or composition can be dissolved in a pharmaceutically effective carrier and be injected into the vitreous of the eye with a fine gauge hollow bore needle (e.g., 30 gauge, 1/2 or 3/8 inch needle) using a temporal approach (e.g., about 3 to about 4 mm posterior to the limbus for human eye to avoid damaging the lens).

In an embodiment, a compound of formula (I) or a pharmaceutically acceptable salt, solvate or prodrug thereof may be formulated in a saline solution and injected into the vitreous of the eye. In a preferred embodiment, the compound of formula (I) or a pharmaceutically acceptable salt, solvate or prodrug is a disodium phosphate ester of the compound formula (I), and 0.1 ng to 10 mg of the disodium phosphate ester of the compound of formula (I) is formulated in a 0.9% saline solution and injected directly into the vitreous of the eye.

A person skilled in the art will appreciate that other means for injecting and/or administering the compound or compositions to the vitreous of the eye can also be used. These other means can include, for example, intravitreal medical delivery devices disclosed US 6,251,090, and US 6,299,603. These devices and methods can include, for example, intravitreal medicine delivery devices, and biodegradable polymer delivery members that are inserted in the eye for long term delivery of medicaments, such as disclosed in US 2005/0250737. These devices and methods can further include transscleral delivery devices, such as disclosed in US 2005/0208103, US 2005/0074497, and US 2005/0064010.

Although intravitreal administration is likely to be the preferred form of administration, other modes of administration including topical or intravenous administration are not excluded. For example, solutions or suspensions of the compound or compositions for use according to the invention may be formulated as eye drops, or as a membranous ocular patch, which is applied directly to the surface of the eye. Topical application typically involves administering the compound of the invention in an amount between 0.1 ng and 10 mg.

The compound or compositions for use according to the invention may also be suitable for intravenous administration. For example, a compound of formula (I) or a pharmaceutically acceptable salt, solvate or C-7 disodium phosphate ester prodrug thereof may be administered intravenously at a dose of up to 16 mg/m².

The compound or compositions for use according to the invention may also be suitable for oral administration and may be presented as discrete units such as capsules, sachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g inert diluent, preservative disintegrant (e.g. sodium starch glycolate, cross-linked polyvinyl pyrrolidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach.

The compound and compositions for use according to the invention may be suitable for topical administration in the mouth including lozenges comprising the active ingredient in a flavoured base, usually sucrose and acacia or tragacanth gum; pastilles comprising the active ingredient in an inert basis such as gelatine and glycerin, or sucrose and acacia gum; and mouthwashes comprising the active ingredient in a suitable liquid carrier.

The compound and compositions for use according to the invention may be suitable for topical administration to the skin may comprise the compounds dissolved or suspended in any suitable carrier or base and may be in the form of lotions, gel, creams, pastes, ointments and the like. Suitable carriers include mineral oil, propylene glycol, polyoxyethylene, polyoxypropylene, emulsifying wax, sorbitan monostearate, polysorbate 60, cetyl esters wax, cetearyl alcohol, 2-octyldodecanol, benzyl alcohol and water. Transdermal patches may also be used to administer the compounds for use according to the invention.

The compound and compositions for use according to the invention may be suitable for parenteral administration include aqueous and non-aqueous isotonic sterile injection solutions which may contain anti-oxidants, buffers, bactericides and solutes which render the composition isotonic with the blood of the intended recipient; and aqueous and non-aqueous sterile suspensions which may include suspending agents and thickening agents. The compositions may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage compositions are those containing a daily dose or unit, daily sub-dose, as herein above described, or an appropriate fraction thereof, of the active ingredient.

It should be understood that in addition to the active ingredients particularly mentioned above, the compositions for use according to this invention may include other agents conventional in the art having regard to the type of composition in question, for example, those suitable for oral administration may include such further agents as binders, sweeteners, thickeners, flavouring agents disintegrating agents, coating agents, preservatives, lubricants and/or time delay agents. Suitable sweeteners include sucrose, lactose, glucose, aspartame or saccharine. Suitable disintegrating agents include cornstarch, methylcellulose, polyvinylpyrrolidone, xanthan gum, bentonite, alginic acid or agar. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Certain embodiments of the invention will now be described with reference to the following examples which are intended for the purpose of illustration only and are not intended to limit the scope of the generality hereinbefore described.

### Examples

### Synthetic Protocols

### Preparation of 2-Bromo-7-acetoxy-3-(3,4,5-trimethoxybenzoyl)-6-methoxybenzofuran.

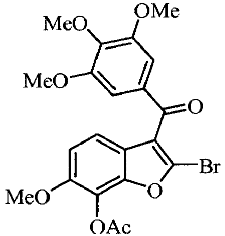

### Step 1: 2-t-Butyldimethylsilyl-3-(t-butyldimethylsilyloxymethylene)-6-methoxy-7-isopropoxybenzofuran (Larock coupling)

A suspension of 2-isopropoxy-3-methoxy-5-iodophenol (4.41 mmol), 1-(*tert*butyldimethylsilyl)-3-(*tert*-butyldimethylsilyloxy)propyne (1.5 g, 5.28 mmol), lithium chloride (189 mg, 4.45 mmol) and sodium carbonate (2.34 g, 22.08 mmol) in dry dimethylformamide (5 mL) at 100 °C was deoxygenated 4 times by evacuation and backfilling with nitrogen. Palladium acetate (135 mg, 0.60 mmol) was added and the reaction vessel was degassed twice with nitrogen. The reaction mixture was then stirred at this temperature for 4 hours (tlc) and the solvent was removed by distillation under vacuum. The residue was dissolved in ethyl acetate (75 mL), stirred well, filtered and treated with triethylamine (5 mL). The solution was concentrated onto silica gel (10 g) and purified by flash chromatography (silica gel, eluent = hexane/diethyl ether/triethylamine; 95:5:1%) to afforded the title compound as a yellow oil (1.45 g, 96 %); ¹H NMR (300 MHz, CDCl₃) δ 7.24(d, 1H, *J* = 8.45 Hz), 6.88(d, 1H, *J* = 8.47 Hz), 4.80(s, 2H, CH₂), 4.73(m, 1H), 3.88(s, 3H, OMe), 1.36(d, 6H, J= 6.17 Hz), 0.94(s, 9H), 0.92(s, 9H), 0.35(s, 6H), 0.12(s, 6H).

### Step 2: 2-t-Butyldimethylsilyl-3-formyl-6-methoxy-7-isopropoxybenzofuran

To a solution of 2-*t*-butyldimethylsilyl-3-(*t*-butyldimethylsilyloxymethylene)-6-methoxy-7-isopropoxybenzofuran (2.69 mmol) in methanol (100 mL) was added concentrated hydrochloric acid (200 µL) and the reaction was stirred for 30 minutes (monitored by tlc), quenched with triethylamine (2 mL) and the solvent removed by distillation under vacuum. The residue was dissolved in dichloromethane (20 mL), washed with water (10 mL), dried over magnesium sulfate, concentrated under vacuum and co-distilled with toluene (20 mL). The crude product was dissolved in dry dichloromethane (4 mL) and added to a stirred solution of Collin's reagent (chromium trioxide (1.01 g), pyridine (1.65 mL) in dry dichloromethane (30 mL)). The suspension was stirred for 10 minutes, filtered and the residue washed with diethyl ether (20 mL). The filtrate was concentrated onto silica (10 g) and purified by flash chromatography (silica gel, eluent = hexane/diethyl-ether/triethylamine (90:9:1) to afford the title compound as a light yellow oil (503 mg, 48%); ¹H NMR (300 MHz, CDCl₃) δ 10.25(s, 1H, CHO), 7.79(d, 1H, *J* = 8.45 Hz), 6.98(d, 1H, *J =* 8.46 Hz), 4.65(m, 1H), 3.89(s, 3H, OMe), 1.35(d, 6H, *J =* 6.17 Hz), 0.97(s, 9H), 0.45(s, 6H).

### Step 3: 2-t-Butyldimethylsilyl-3-(3,4,5-trimethoxybenzoyl)-6-methoxy-7-isopropoxybenzofuran

To a stirred solution of 3,4,5-trimethoxyiodobenzene (377 mg, 1.27 mmol) in dry tetrahydrofuran (1 mL) at -78 °C under nitrogen was added n-butyllithium (795 µL, 1.59 mmol, 2M solution in cyclohexane) and the reaction mixture was stirred at this temperature for 40 minutes. After this time a solution of 2-t-butyldimethylsilyl-3-formyl-6-methoxy-7-isoproxybenzofuran (1.07 mmol) in dry tetrahydrofuran (1 mL) was added to the reaction dropwise via syringe pipette. The reaction mixture was stirred at -60 °C for 20 minutes and then allowed to warm to 0°C, stirred for 10 minutes, quenched with saturated ammonium chloride solution (2 mL) and diluted with ethyl acetate (20 mL). The organic layer was washed with water (10 mL), dried over magnesium sulfate and the solvent was removed under vacuum to give a residue that was co-distilled with toluene. The crude product (908 mg) was dissolved in dry tetrahydrofuran (10 mL) and treated with 2,3-dichloro-5,6-dicyano-1,4-benzoquinone (900 mg, 1.59 mmol) was added. The reaction mixture was stirred at room temperature for 16 hours (monitored by tlc) and then loaded onto silica (10 g) and purified by flash chromatography (silica gel, eluent = hexane/diethyl ether/triethylamine, 90:9:1) to afford the title compound as a light yellow oil (498 mg, 69%); ¹H NMR (300 MHz, CDCl₃) δ 7.14(s, 2H, benzoyl Hs), 6.81(d, 1H, *J=* 8.64 Hz), 6.77(d, 1H, *J=* 8.64 Hz) 4.74(m, 1H), 3.93(s, 3H, OMe), 3.86(s, 3H, OMe), 3.78(s, 6H, 2 x OMe), 1.39(d, *6H, J=* 6.14 Hz), 1.01(s, 9H), 0.26(s, 6H).

### Step 4: 2-(tert-butyldimethylsilyloxy)-7-acetoxy-3-(3,4,5-trimethoxybenzoyl)-6-methoxybenzofuran

To a stirred solution of 2-(*t*-butyldimethylsilyloxy)-7-isopropoxy-3-(3,4,5-trimethoxybenzoyl)-6-methoxy-benzofuran (160 mg, 0.31 mmol) in dry DCM (2 mL) at room temperature under nitrogen was added solid aluminium trichloride (83 mg, 0.62 mmol) and the reaction mixture was stirred for 15 minutes (monitored by tlc). The reaction was quenched with a saturated solution of ammonium chloride, extracted with dichloromethane and dried over magnesium sulfate. The solvent was removed by distillation and residue was dried by azeotropic removal of water with toluene. The crude product was dissolved in pyridine (2 mL), acetic anhydride (1 mL) was added and reaction mixture was stirred for 2 hours at room temperature. The solvent was distilled under vacuum and the residue was loaded onto silica gel (1 g) and purified by column chromatography (silica gel, eluent, hexane:diethyl-ether; 80:20) (134 mg, 84%); ¹H NMR (300 MHz, CDCl₃) δ 7.14(s, 2H, benzoyl Hs), 6.98(d, 1H, *J =* 8.72 Hz), 6.85(d, 1H, *J =* 8.72 Hz), 3.93(s, 3H, OMe), 3.86(s, 3H, OMe), 3.80(s, 6H, 2 x OMe), 2.41(s, 3H), 0.99(s, 9H), 0.25(s, 6H).

### Step 5: 2-Bromo-7-acetoxy-3-(3,4,5-trimethoxybenzoyl)-6-methoxybenzofuran

To a stirred solution of 2-t-butyldimethylsilyl-7-acetoxy-3-(3,4,5-trimethoxybenzoyl)-6-methoxybenzofuran (120 mg, 0.44 mmol) in 1,2-dichloroethane (1 mL) at room temperature under nitrogen was added bromine (12 µl, 0.44 mmol) dropwise and the reaction mixture was stirred at this temperature for 10 minutes. After this time the reaction was quenched with saturated sodium thiosulfate solution, extracted with ethyl acetate (20 mL), dried over magnesium sulfate and the solvent removed by distillation under vacuum. The crude product was purified by silica gel column chromatography (eluent = Hexane:diethyl ether; 8:2 - 7:3) to afford the title compound as a colourless crystalline solid (91 mg, 81 %); ¹H NMR (300 MHz, CDCl₃) δ 7.40(d, 1H, *J =* 8.70 Hz), 7.14(s, 2H, benzoyl-Hs), 6.98(d, 1H, *J* = 8.75 Hz), 3.94(s, 3H, OMe), 3.89(s, 3H, OMe), 3.86(s, 6H, 2 x OMe), 2.43(s, 3H); ¹³C NMR (75 MHz, CDCl₃) δ 187.95(CO), 167.71, 152.75, 149.54, 147.49, 142.59, 131.92, 131.80, 123.91, 121.84, 119.89, 117.72, 109.89, 106.92, 60.69, 56.61, 56.00, 20.09.

### Example 1

### Preparation of 2-Methyl-7-hydroxy-3-(3,4,5-trimethoxybenzoyl)-6-methoxybenzofuran

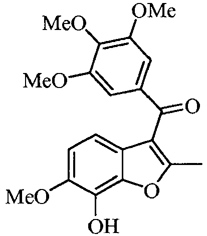

### Preparation A

To a stirred solution of 2-Bromo-7-acetoxy-3-(3,4,5-trimethoxybenzoyl)-6-methoxybenzofuran (20 mg, 0.042 mmol), methyl-boronic acid (40 mg, 0.67 mmol), in 1,4-dioxane (2 mL) at 90 °C was added *tetrakis*-triphenylphosphine palladium (11 mg, 0.01 mmol) followed by the addition of a solution of sodium bicarbonate (40 mg, 0.48 mmol) in distilled water (0.5 mL). The reaction mixture turned red after 5 minutes. After 2 hours (tlc) the reaction mixture was brought to room temperature and was added saturated ammonium chloride (2 mL) and diluted with dichloromethane (20 mL). The organic layer was separated and washed with water, dried over magnesium sulfate and the solvent was removed by distillation under vacuum. The residue was purified by PTLC (eluent = Dichloromethane/Methanol, 1:1) to give the title compound (actate cleaved during rection) as a fluffy white solid; (3 mg, 19%).

### Preparation B (Negishi Coupling)

To a stirred solution of zinc-bromide (592 mg, 2.63 mmol) in dry THF(1.5 mL) at 0°C was added the solution of methyl lithium (1.6 M solution in diethyl-ether, 2.6 mL, 4.15 mmol) and the reaction mixture was stirred for 2 hours. Solid 2-bromo-7-acetoxy-3-(3,4,5-trimethoxybenzoyl)-6-methoxy-benzofuran (300 mg, 0.63 mmol) was added and the ether was removed under vacuum and to the rest suspension was added dichlorobis(triphenylphosphine)palladium catalyst (21 mg) and catalytic amount of copper (I) iodide. The reaction mixture was stirred at room temperature for 36 hours (monitored by tlc), quenched with saturated ammonium chloride solution and extracted with dichloromethane (10 mL), dried over magnesium sulfate and solvent distilled under vacuum and the product was purified by silica gel column (eluent = hexane/ethyl acetate; 8:2). The product was crystallized in methanol (106 mg, 46%); ¹H NMR (300 MHz, CDCl₃) δ 7.09(s, 2H, benzoyl Hs), 6.93(d, 1H, *J* = 8.54 Hz), 6.83(d, 1H, *J* = 8.56 Hz), 5.70(bs, 1H, OH), 3.93(s, 3H, OMe), 3.92(s, 3H, OMe), 3.83(s, 6H, 2 x OMe), 2.54(s, 3H, 2-Me)

### Example 2

### Preparation of Disodium 6-methoxy-2-methyl-3-(3,4,5-trimethoxybenzoyl)benzofuran-7-yl phosphate

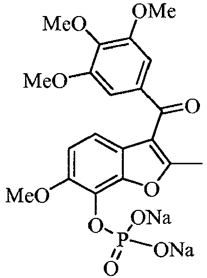

### Step 1: Dibenzyl 6-methoxy-2-methyl-3-(3,4,5-trimethoxybenzoyl)benzofuran-7-yl phosphate:

To a mixture of 0.081 g (0.22 mmol) of (7-hydroxy-6-methoxy-2-methylbenzofuran-3-yl)(3,4,5-trimethoxyphenyl)methanone, 0.086 g (0.261 mmol) of carbon tetrabromide and 0.063 ml (0.283 mmol) of dibenzylphosphite in 2.5 ml of anhydrous acetonitrile 0.046 ml of anhydrous triethylamine was added dropwise at 0°C under nitrogen atmosphere. The resulting mixture was stirred for 2h at room temperature, then diluted to 20 ml with ethyl acetate, washed with water brine, dried over anhydrous magnesium sulfate, filtered off and evaporated to dryness under reduced pressure. The residue was purified by flash column chromatography (dichloromethane/ ethyl acetate, 9:1) to give the title compound as a colorless foam (0.13g, 94%); ¹H NMR (CDCl₃) δ 2.42 (s, 3H, Me-2); 3.83 (s, 1H, OMe); 3.93 (s, 3H, OMe); 5.33 (m, 4H, CH₂Ph); 6.89 (d, CH aromatic, *J* = 8.7 Hz); 7.21 (dd, 1H, CH aromatic, *J* = 8.72 Hz; *J =* 1.2 Hz); 7.08 (s, 2H, CH aromatic); 7.29 - 7.43 (m, 10 H, CH aromatic).

### Step 2: Disodium 6-methoxy-2-methyl-3-(3,4,5-trimethoxybenzoyl)benzofuran-7-yl phosphate:

To a stirred solution of 0.122 g (0.193 mmol) of the product from Step 1 in 1 ml of anhydrous acetonitrile 0.075 ml (0.58 mmol) of bromotrimethylsilane was added at -5°C under nitrogen atmosphere. The resulting mixture was stirred for 1 h at 0°C, then evaporated to dryness *in vacuo.* The residue was diluted to 5 ml with anhydrous methanol and pH of the solution was brought up about 10 by the addition of sodium methoxide. After evaporation of the resulting mixture under reduced pressure the solid residue was washed with anhydrous isopropanol (4 x 1.5 ml) and anhydrous ethanol (3 x 1.5 ml) and dried under vacuum to give 0.062 g (65 % yield) of title compound as an colorless solid; ¹H NMR (D₂O) δ 2.37 (s, 3H, Me-2); 3.76 (s, 6H, OMe); 3.79 (s, 3H, OMe); 3.82 (s, 3H, OMe); 4.66 (s, H₂O); 6,93 (d, 1H, CH aromatic, *J* = 8.6 Hz); 7.04 (d, 1H, CH aromatic, *J* = 8.6 Hz); 7.10 (s, 2H, CH aromatic).

### Biological Data

### General Description of Biological Experiments

**Tubulin Polymerisation Assay:** Tubulin polymerisation inhibition assays were performed using a fluorescent-based detection kit (#BK011, Cytoskeleton) according to the instructions of the manufacturer. The test compound was added to a 2mg/ml tubulin solution containing 20% glycerol and 1mM GTP in 1x Buffer 1 (Buffer 1: 80mM Piperazine-N,N'-bis[2-ethanesulfonic acid] sequisodium salt; 2mM magnesium chloride; 0.5mM Ethylene glycol-bis(b-amino-ethyl ether)N,N,N',N'-tetra-acetic acid, pH 6.9, 10uM fluorescent reporter). Fluorescence was measured over a period of 42 minutes at 1 minute intervals. Increased fluorescence indicates increase in tubulin polymerisation. There is a ten-fold increase in the affinity of the fluorescent reporter for polymerised tubulin compared to monomeric tubulin subunits. The result is a fluorescence signal that closely follows tubulin polymerisation.

**Proliferation Assay - quiescent endothelium:** Human umbilical vein endothelial cells (CC-2519, Clonetics) were plated at 15000 cells/well in EBM2 (CC-3156, Clonetics) + 0.5%FBS (CC-4101A, Clonetics) + GA-1000 (CC-4381A, Clonetics) in a 96 well plate in triplicate. Cells were cultured overnight at 37°C 5% CO₂. Medium was subsequently replaced with fresh medium including the compound or negative control. Cells were cultured for a period of 48hrs. An MTT assay was performed to measure changes in cell numbers. Briefly, 20µl of MTT reagent was added to cells containing 100µl of EBM2 + 0.5%FBS and incubated at 37°C for 2 hours. Absorbance was measured at 492nm.

**Proliferation Assay - activated endothelium:** Human umbilical vein endothelial cells (CC-2519, Clonetics) were plated at 2500 cells/well in EGM2 (CC-3162, Clonetics) in a 96 well plate in triplicate. Cells were cultured overnight at 37°C 5% CO₂. Medium was subsequently replaced with fresh medium including the compound or negative control. Cells were cultured for a period of 48hrs. An MTT assay was performed to measure changes in cell numbers. Briefly, 20µl of MTT reagent was added to cells containing 100µl of EGM2 and incubated at 37°C for 2 hours. Absorbance was measured at 492nm.

### Results

### Inhibition of endothelial cell proliferation

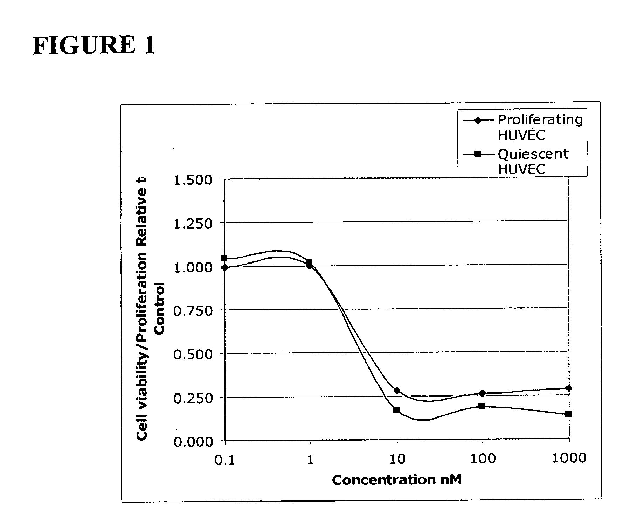

The selectivity of the compound of the invention for angiogenic endothelium is a critical determinant of its potential therapeutic utility for macular degeneration. Normal endothelial cells, found in healthy tissues, are in a "quiescent" state while endothelial cells involved with age-related macular degeneration take on an "activated" angiogenic/proliferative state. Most TPIs or therapeutic targets do not distinguish between <these two states. For example Combretastatin (CA4) is equally potent against quiescent and activated endothelial cells (refer to Figure 1 and Table 2 below).

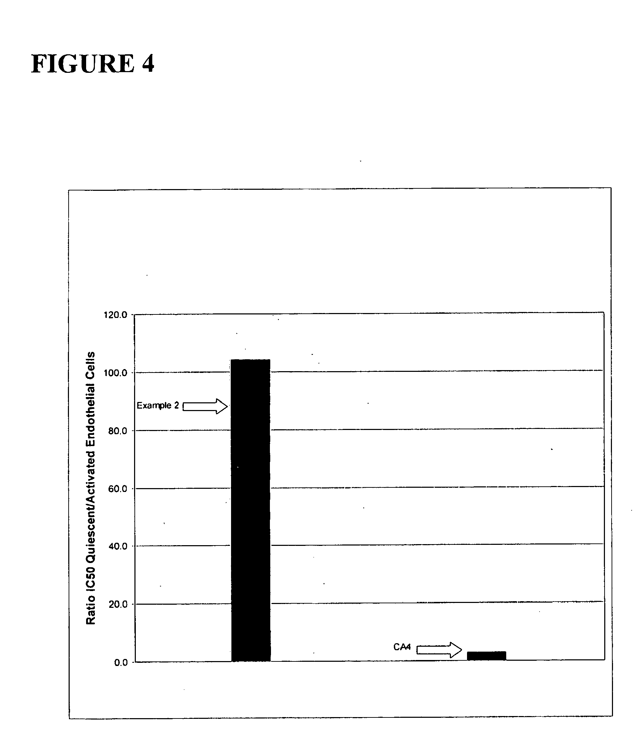

The ability of compound Example 2 to selectively display cytotoxic activity for actively proliferating endothelial (HUVEC) cells as compared to CA4 is shown in Figure 4. Cytotoxic activity was tested in activated (angiogenic) endothelial cells as compared to activity against quiescent (non-angiogenic) cells. The ratio for IC50 activity in terms of quiescent/activated HUVEC endothelial cells in culture is measured. These data show that compound Example 2 is significantly more selective than CA4 in inhibiting proliferation of activated endothelial cells as compared to quiescent endothelial cells. This was also true for similarly structured benzofuran based compounds (data not shown).

In addition, the data presented in Table 2 below shows the selectivity ratio for compound Example 2 and CA4 against proliferating and quiescent endothelial cells. These data clearly indicate that compound Example 2 is significantly more selective for activated (angiogenic/proliferating) endothelial cells as compared to quiescent cells in both human abdominal aorta endothelial cell (HAAEC) and human umbilical vein endothelial cell (HUVEC) cell types.

| Table 2 | Selectivity ratio for compound Example 2 and CA4 against proliferating and quiescent endothelial cells. | | |
|---|---|---|---|
| **Compound** | **Activated EC₅₀** | **Quiescent EC₅₀** | **Selectivity Ratio Quiescent/Activated** |
| | ***HUVEC*** | | |
| Example 2 | 0.14 nM | 23.3 nM | 166 |
| CA4 | 5.8 nM | 2.3 nM | 0.4 |

| | ***HAAEC*** | | |
|---|---|---|---|
| Example 2 | 0.1 nM | 12.5 nM | 125 |
| CA4 | 2.2 nM | 1.3 nM | 0.6 |

| | | | |
|---|---|---|---|
| Abbreviations: CA4, Combretastatin; EC₅₀, 50% efficacious concentration; HAAEC, human abdominal aorta endothelial cells; HUVEC, human umbilical vein endothelial cells | | | |

### Anti-angiogenic activity in vitro

### (i) Effect on capillary formation

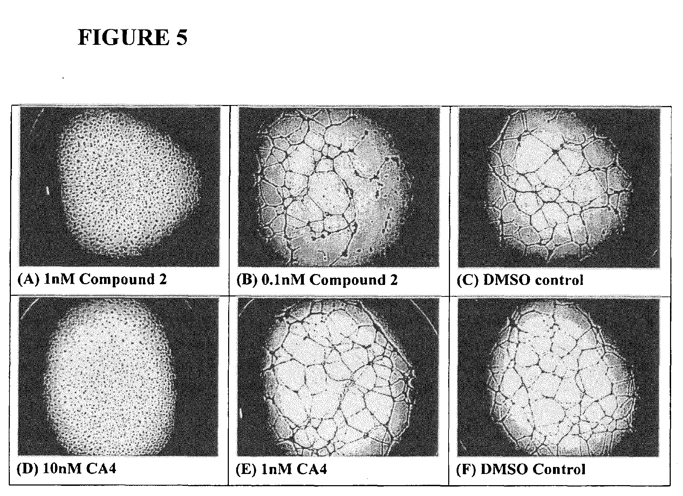

Figure 5 shows that HUVEC endothelial cell capillary formation on Matrigel was inhibited by compound Example 2 of the invention (A, B). The effect on capillary formation was more significant than CA4 (D, E). DMSO vehicle (C, F) was included in the analysis as a negative control. Cells were plated at 25,000/well in 96-well plates and cultured for 22hrs.

### (ii) Disruptive activity on existing capillaries

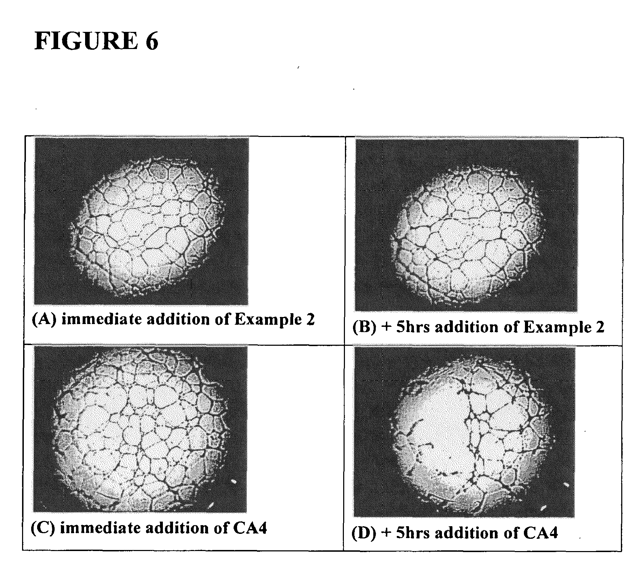

The disruptive activity exhibited by compound Example 2 and CA4 on stable pre-formed HUVEC capillary tubes on Matrigel® was next examined. Although compound Example 2 suppresses the formation of new capillaries (ie. it has an anti-angiogenic effect; Figure 5) Figure 6 shows that compound Example 2 does not disrupt stable pre-formed capillaries (Figure 6A and 6B), whereas CA4 was shown to disrupt stable preformed capillaries (Figure 6C and 6D). In these experiments, pre-formed HUVEC capillaries were exposed to compound Example 2 and CA4 concentrations that were found to inhibit capillary formation (Figure 5). These data clearly demonstrate that compound Example 2 has low activity against normal stable blood vessels.

### Compound Example 2 disrupts angiogenesis by disrupting endothelial cell cytoskeleton

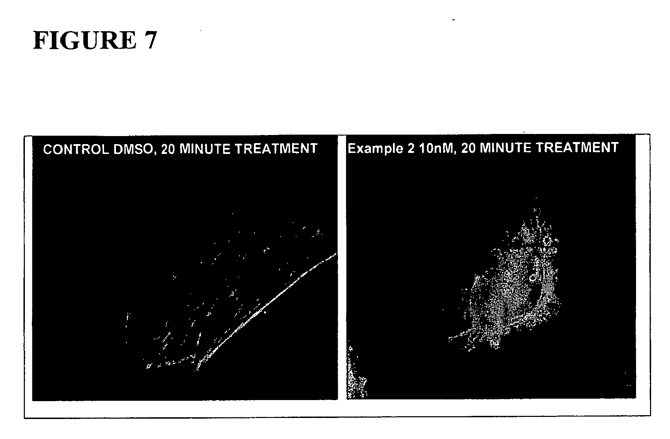

Figure 7 shows that compound Example 2 inhibits angiogenesis by disrupting the endothelial cell cytoskeleton. HUVEC cells were cultured under "activated" cell conditions in the presence of 10nM compound Example 2 or DMSO control (using HUVEC cells). After 20 minutes cells were washed, fixed and stained for actin. Compound Example 2 treated cells displayed clear evidence of "blebbing". A bleb is defined as an irregular bulge in the plasma membrane of a cell caused by localized decoupling of the cytoskeleton from the plasma membrane. Blebbing is the term used to describe the formation of blebs.

## Claims

1. A pharmaceutical composition comprising an effective amount of a compound of formula (I) or a pharmaceutically acceptable salt, solvate or C-7 disodium phosphate ester prodrug thereof: 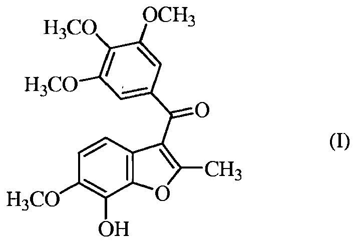 optionally in combination with a pharmaceutically acceptable carrier, excipient or diluent, for use in the treatment of macular degeneration (MD).

2. A pharmaceutical composition for use according to claim 1 wherein the composition additionally includes a vascular endothelial growth factor (VEGF) inhibitor as a second therapeutic agent.

3. A pharmaceutical composition for use according to claim 2 wherein the VEGF inhibitor is avastin, lucentis or macugen.

4. A use of a compound of formula (I) or a pharmaceutically acceptable salt, solvate or C-7 disodium phosphate ester prodrug thereof: 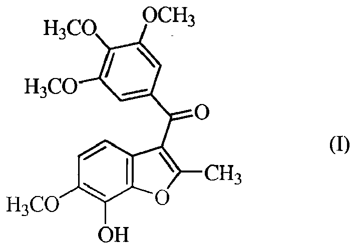 in the manufacture of a medicament for treating macular degeneration (MD).

5. Use according to claim 4 wherein the medicament additionally includes a vascular endothelial growth factor (VEGF) inhibitor as a second therapeutic agent.

6. Use according to claim 5 wherein VEGF inhibitor is avastin, lucentis or macugen.

7. A compound of formula (I) or a pharmaceutically acceptable salt, solvate or C-7 disodium phosphate ester prodrug thereof: 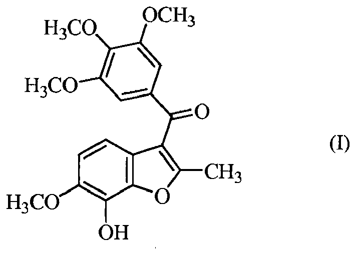 for use in the treatment of macular degeneration (MD).

8. A composition or compound for use according to any of claims 1 to 3 or 7 or the use according to any one of claims 4 to 6 wherein the compound of formula (I) is represented as:

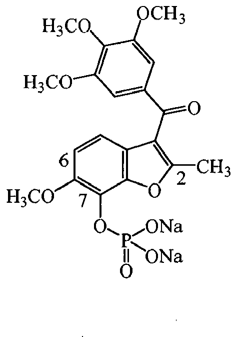

## Patentansprüche

1. Pharmazeutische Zusammensetzung, umfassend eine wirksame Menge einer Verbindung der Formel (I) oder eines pharmazeutisch unbedenklichen Salzes, Solvats oder C-7 Dinatriumphosphatester-Pro-Pharmakons davon: 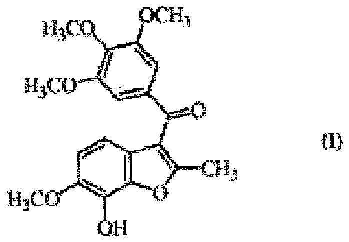 gegebenenfalls in Kombination mit einem pharmazeutisch unbedenklichen Träger, Exzipienten oder Verdünnungsmittel zur Verwendung bei der Behandlung von Makuladegeneration (MD).

2. Pharmazeutische Zusammensetzung zur Verwendung nach Anspruch 1, wobei die Zusammensetzung zusätzlich einen Gefäßendothelwachstumsfaktor (VEGF)-Inhibitor als zweites therapeutisches Mittel umfasst.

3. Pharmazeutische Zusammensetzung zur Verwendung nach Anspruch 2, wobei der VEGF-Inhibitor Avastin, Lucentis oder Macugen ist.

4. Verwendung einer Verbindung der Formel (I) oder eines pharmazeutisch unbedenklichen Salzes, Solvats oder C-7 Dinatriumphosphatester-Pro-Pharmakons davon: 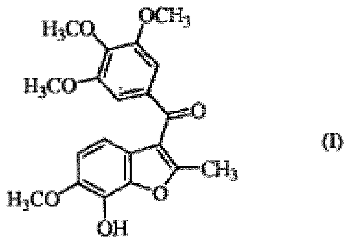 bei der Herstellung eines Medikaments zur Behandlung von Makuladegeneration (MD).

5. Verwendung nach Anspruch 4, wobei das Medikament zusätzlich einen Gefäßendothelwachstumsfaktor (VEGF)-Inhibitor als zweites therapeutisches Mittel umfasst.

6. Verwendung nach Anspruch 5, wobei der VEGF-Inhibitor Avastin, Lucentis oder Macugen ist.

7. Verbindung der Formel (I) oder pharmazeutisch unbedenkliches Salz, Solvat oder C-7 Dinatriumphosphatester-Pro-Pharmakon davon: 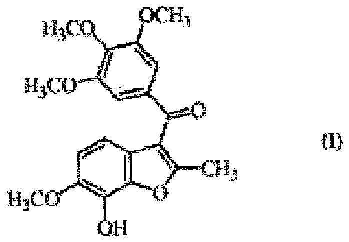 zur Verwendung bei der Behandlung von Makuladegeneration (MD).

8. Zusammensetzung oder Verbindung zur Verwendung nach einem der Ansprüche 1 bis 3 oder 7 oder zur Verwendung nach einem der Ansprüche 4 bis 6, wobei die Verbindung der Formel (I) dargestellt wird als:

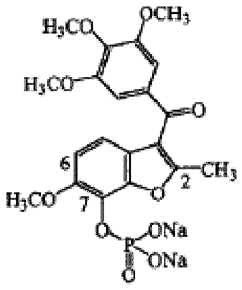

## Revendications

1. Composition pharmaceutique comprenant une quantité efficace d'un composé de formule (I) ou d'un sel pharmaceutiquement acceptable, solvate, ou promédicament ester phosphate disodique en C-7 d'un tel composé : 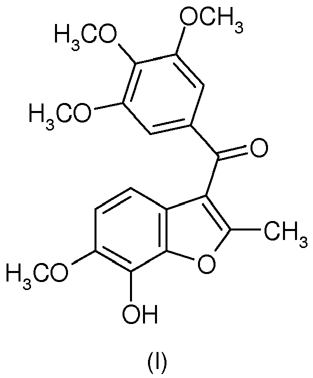 éventuellement en combinaison avec un véhicule, excipient ou diluant pharmaceutiquement acceptable, pour utilisation dans le traitement de la dégénérescence maculaire (MD).

2. Composition pharmaceutique pour utilisation selon la revendication 1, laquelle composition contient de plus un inhibiteur de facteur de croissance endothélial vasculaire (VEGF) à titre de deuxième agent thérapeutique.

3. Composition pharmaceutique pour utilisation selon la revendication 2, dans laquelle l'inhibiteur de VEGF est l'avastine, le lucentis ou le macugen.

4. Utilisation d'un composé de formule (I) ou d'un sel pharmaceutiquement acceptable, solvate, ou promédicament ester phosphate disodique en C-7 d'un tel composé : 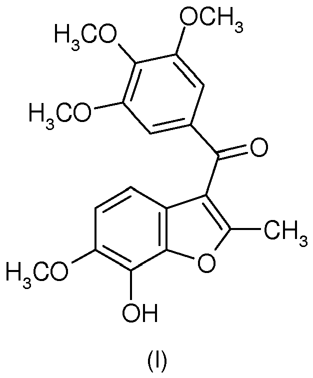 dans la fabrication d'un médicament pour traiter la dégénérescence maculaire (MD).

5. Utilisation selon la revendication 4, dans laquelle le médicament contient de plus un inhibiteur de facteur de croissance endothélial vasculaire (VEGF) à titre de deuxième agent thérapeutique.

6. Utilisation selon la revendication 5, dans laquelle l'inhibiteur de VEGF est l'avastine, le lucentis ou le macugen.

7. Composé de formule (I) ou un sel pharmaceutiquement acceptable, solvate, ou promédicament ester phosphate disodique en C-7 d'un tel composé : 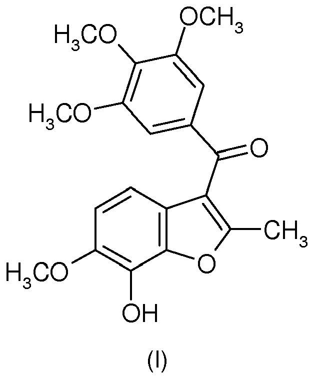 pour utilisation dans le traitement de la dégénérescence maculaire (MD).

8. Composition ou composé pour utilisation selon l'une quelconque des revendications 1 à 3 et 7 ou utilisation selon l'une quelconque des revendications 4 à 6, dans laquelle le composé de formule (I) est représenté par :
